# EUROPÄISCHE PATENTSCHRIFT

(11) **EP 2 726 455 B1**
(45) Veröffentlichungstag und Bekanntmachung des Hinweises auf die Patenterteilung: **12.10.2016**
(21) Anmeldenummer: 12725429.0
(22) Anmeldetag: 04.06.2012
(51) Int. Cl.: C07C 403/24

(54) **PHOTOLABILE DUFTSPEICHERSTOFFE**
PHOTOLABILE PRO-FRAGRANCES
SUBSTANCES PHOTOLABILES RENFERMANT UN PARFUM

(30) Priorität: 30.06.2011 DE 102011078416
(43) Veröffentlichungstag der Anmeldung: 07.05.2014
(73) Patentinhaber: Henkel AG & Co. KGaA, 40589 Düsseldorf (DE)
(72) Erfinder: GERKE, Thomas, 40627 Düsseldorf (DE); KROPF, Christian, 40724 Hilden (DE); HUCHEL, Ursula, 50733 Köln (DE); GRIESBECK, Axel, 50937 Köln (DE); HINZE, Olga, 50679 Köln (DE)
(86) Internationale Anmeldenummer: PCT/EP2012/060471
(87) Internationale Veröffentlichungsnummer: WO 2013/000668

(56) Entgegenhaltungen:
- WO-A2-01/96272
- WO-A2-02/30860
- KATRITZKY ALAN R ET AL: "Polymers by the reaction of bis(pyrylium salts) with diamines: a novel approach to ionene polymers", JOURNAL OF POLYMER SCIENCE. PART A, POLYMER CHEMISTRY, Bd. 26, Nr. 12, 1. Januar 1988 (1988-01-01), Seiten 3323-3336, XP002462919, JOHN WILEY & SONS, INC, US ISSN: 0887-624X, DOI: 10.1002/POLA.1988.080261217
- G.D. PARKES ET AL.: "The Preparation of p-Di-(6-carboxyhexanoyl)benzene", J. CHEM. SOC., 1955, Seite 3294, XP002679491,
- DONALD S. ACKER: "Syntheses of Reduced Lipoic Acid and Analogs of Lipoic Acid", J. ORG. CHEM., 1963, Seiten 2533-2536, XP002679492,
- YOSUKE ITO ET AL.: "One-pot synthesis of cyclophane-type macrocycles using manganese(III)-mediated oxidative radical cyclization", ORGANIC & BIOMOLECULAR CHEMISTRY, Bd. 9, Nr. 5, 7. März 2011 (2011-03-07), Seiten 1491-1507, XP002679493, DOI: 10.1039/c0ob00683a -& YOSUKE ITO ET AL.: "Supplementary data: One-pot synthesis of cyclophane-type macrocycles usingmanganese(III)-mediated oxidative radical cyclization", Organic & Biomolecular Chemistry , 7. März 2011 (2011-03-07), Seiten 1-11, XP002682822, Gefunden im Internet: URL:http://www.rsc.org/suppdata/ob/c0/c0ob 00683a/c0ob00683a.pdf
- DATABASE CA [Online] CHEMICAL ABSTRACTS SERVICE, COLUMBUS, OHIO, US; XP002679494, Database accession no. 85:192312

## Beschreibung

Die vorliegende Erfindung betrifft spezielle Ketone, die als photolabile Duftspeicherstoffe fungieren. Ferner betrifft die vorliegende Erfindung Wasch- oder Reinigungsmittel, welche solche Ketone enthalten. Ferner betrifft sie ein Verfahren zur lang anhaltenden Beduftung von Oberflächen.

Wasch- oder Reinigungsmittel enthalten zumeist Duftstoffe, die den Mitteln einen angenehmen Geruch verleihen. Die Duftstoffe maskieren dabei zumeist den Geruch der anderen Inhaltstoffe, so dass beim Verbraucher ein angenehmer Geruchseindruck entsteht.

Insbesondere im Bereich Waschmittel sind Duftstoffe (synonymer Ausdruck: Riechstoffe) wichtige Bestandteile der Zusammensetzung, da die Wäsche sowohl im feuchten als auch im trockenen Zustand einen angenehmen und möglichst auch frischen Duft aufweisen soll. Man steht beim Einsatz von Duftstoffen grundsätzlich vor dem Problem, dass es sich bei diesen um mehr oder minder leicht flüchtige Verbindungen handelt, aber dennoch ein lange anhaltender Dufteffekt angestrebt wird. Insbesondere bei denjenigen Riechstoffen, die die frischen und leichten Noten des Parfüms darstellen und infolge ihres hohen Dampfdruck besonders schnell abdampfen, ist die gewünschte Langlebigkeit des Dufteindrucks kaum erreichbar.

Eine verzögerte Duftfreisetzung kann z.B. durch Träger-gebundenen Einsatz von Duftstoffen erfolgen. Eine Träger-gebundene Vorform eines Duftstoffes wird auch als "ProFragrance" oder Duftspeicherstoff bezeichnet. In diesem Zusammenhang offenbart die internationale Patentanmeldung WO2007/087977 die Verwendung von 1-Aza-3,7-dioxabicyclo[3.3.0]octan-Verbindungen als Duftspeicherstoffe zur verzögerten Freisetzung von Duftaldehyden und Duftketonen durch Hydrolyse. Eine alternative Möglichkeit der verzögerten Freisetzung von Duftstoffen stellt der Einsatz von so genannten photoaktivierbaren Substanzen als Duftspeicherstoffe dar. Durch die Einwirkung des Sonnenlichts oder einer anderen elektromagnetischen Strahlungsquelle bestimmter Wellenlänge wird der Bruch einer kovalenten Bindung im Duftspeicherstoff-Molekül induziert, wodurch ein Duftstoff freigesetzt wird.

Das US-Patent 6,949,680 offenbart die Verwendung bestimmter Phenyl- oder Pyridylketone als photoaktivierbare Substanzen, die in Gegenwart von Licht in einer photochemischen Fragmentierung ein terminales Alken als Aktivstoff freisetzen. Der genannte Aktivstoff besitzt beispielsweise eine duftgebende oder antimikrobielle Aktivität, die durch den photochemisch induzierten Zerfall erst verzögert und über einen längeren Zeitraum hinweg auf einer bestimmten Oberfläche freigesetzt wird.

WO2009/118219 A1 beschreibt bestimmte Ketone als photoaktivierbare Substanzen, welche die verzögerte Freisetzung cyclischer Verbindungen mit mindestens einer cyclischen Doppelbindung, insbesondere cyclischer Terpene oder cyclischer Terpenoide mit mindestens einer cyclischen Doppelbindung erlauben.

WO2010/066486 A2 beschreibt bestimmte beta-Hydroxyketone als photoaktivierbare Substanzen, welche in Gegenwart von Licht eine Freisetzungvon Duftaldehyden (Riechstoffaldehyde) und Duftketonen (Riechstoffketone) ermöglichen.

Aufgabe der vorliegenden Erfindung war die Bereitstellung weiterer photoaktivierbarer Substanzen als Duftspeicherstoffe, welche die verzögerte Freisetzung von Riechstoffketonen, insbesondere von Damascone erlauben.

### Gelöst wurde diese Aufgabe durch eine Verbindung der allgemeinen Formel (I),

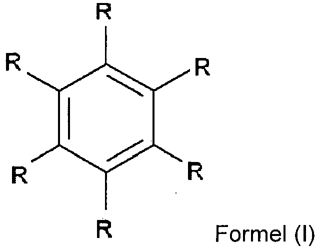

wobei zumindest zwei der Reste R in Formel (I) für den in eckigen Klammern gezeichneten Rest a stehen, 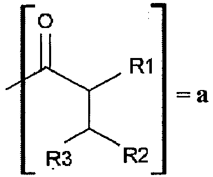 und wobei die übrigen Reste R in Formel (I), unabhängig voneinander, jeweils für Wasserstoff, ein Halogenatom, -NO₂, eine lineare oder verzweigte, substituierte oder unsubstituierte Alkoxygruppe mit 1 bis 15 C-Atomen, eine lineare oder verzweigte, substituierte oder unsubstituierte Alkylgruppe mit 1 bis 15 C-Atomen, einen Arylrest, einen Cycloalkylrest, Acylrest,-OH,-NH-Alkyl,-NH₂ oder -N(Alkyl)₂ stehen,
und wobei für jeden in eckigen Klammern stehenden Rest a, unabhängig voneinander gilt, dass
R2 für einen substituierten Kohlenwasserstoffrest steht, der mindestens eine C=O-Gruppe aufweist,
R1 für eine lineare oder verzweigte, substituierte oder unsubstituierte Alkylgruppe mit 1 bis 6 C-Atomen, insbesondere für eine Methylgruppe steht, und
R3 für Wasserstoff, ein Halogenatom, -NO₂, eine lineare oder verzweigte, substituierte oder unsubstituierte Alkoxygruppe mit 1 bis 15 C-Atomen, eine lineare oder verzweigte, substituierte oder unsubstituierte Alkylgruppe mit 1 bis 15 C-Atomen, einen Arylrest, einen Cycloalkylrest, Acylrest, -OH, -NH-Alkyl, -NH2 oder -N(Alkyl)₂ steht.

Der Rest R2, der für einen substituierten Kohlenwasserstoffrest steht, der mindestens eine C=O-Gruppe aufweist, kann linear oder verzweigt sein, insbesondere kann er auch zumindest ein Ringsystem umfassen.

Bei den erfindungsgemäßen Verbindungen der allgemeinen Formel (I) handelt es sich um Ketone, so dass diese im Folgenden auch als erfindungsgemäße Ketone bzw. als erfindungsgemäße Ketone gemäß Formel (I) bezeichnet werden. Es konnte überraschend gefunden werden, dass die erfindungsgemäßen Ketone besonders wirksame Duftspeicherstoffe sind, welche die verzögerte Freisetzung von Riechstoffketonen, insbesondere von Damascone erlauben. Die Anwendung der erfindungsgemäßen Ketone in Wach-, Reinigungs- oder Pflegemitteln führte bei deren Anwendung zu einer verbesserte Langzeitduftwirkung, insbesondere im Zusammenhang mit der Textilbehandlung. Z.B. konnte bei der Anwendung erfindungsgemäßer Ketone in einem Wäschebehandlungsmittel, wie z.B. Waschmittel sowie Weichspüler, eine verbesserte Langzeitduftwirkung der behandelten Wäsche gefunden werden. Ferner weisen entsprechende Produkte eine besonders gute Lagerstabilität auf. Die erfindungsgemäßen Mittel ermöglichen es zudem, die Gesamtmenge an Parfüm, welche im Mittel enthalten ist, zu reduzieren, und dennoch Geruchsvorteile auf den gewaschenen Textilien zu erzielen, insbesondere mit Blick auf das Frischeempfinden. Besonders vorteilhaft ist weiterhin das günstige Verhältnis von abspaltbaren Riechstoffen zum Ankermolekül. Pro Trägermolekül werden mindestens zwei Moleküle Riechstoff freigesetzt. Dies ermöglicht einen besonders effektiven Einsatz für Beduftungszwecke. Die hervorragende Langzeitduftwirkung scheint dadurch erklärbar, dass bei der Abspaltung der gespeicherten Riechstoffe auf dem Zielsubstrat davon auszugehen ist, dass diese sukzessive verläuft, dass also ein (oder mehrere) gebundenes Riechstoffmolekül noch als Anker wirkt, während ein anderes Riechstoffmolekül bereits abgespalten wird.

Es ist auch möglich, dass einzelne der Reste R des Ketons der allgemeinen Formel (I), untereinander zu Cyclen, z.B. über C, O, N oder S-Atome, verbrückt sind.

Es ist bevorzugt, dass in dem Rest a R3 für Wasserstoff oder eine lineare oder verzweigte, substituierte oder unsubstituierte Alkylgruppe mit 1 bis 6 C-Atomen, insbesondere für eine Methylgruppe steht.

Die Reste a, mit denen der Benzolring in Formel (I) substituiert ist, können jeweils gleich sein oder sie können unterschiedlich sein. In einer bevorzugten Ausführungsform sind dieses Reste a gleich. Diese Ausführungsform ist besonders dann vorteilhaft, wenn ein besonders intensiver Geruch eines bestimmten Riechstoffes erzeugt werden soll. Die Ausführungsform, in welcher unterschiedliche Reste a eingesetzt werden, ist dann besonders vorteilhaft, wenn Mischgerüche angestrebt werden.

Bevorzugt stehen 2 der Reste R in Formel (I) für den in eckigen Klammern stehenden Rest a, es können aber auch mehr sein, z.B. können auch 3 oder 4 der Reste R in Formel (I) für den in eckigen Klammern stehenden Rest a stehen.

Im Falle von 2 Resten R in Formel (I) gemäß dem in eckigen Klammern stehenden Rest a sind diese am Benzolring vorzugsweise in 1,4- Stellung zueinander angeordnet. Im Falle von 3 Resten R in Formel (I) gemäß dem in eckigen Klammern stehenden Rest a sind diese am Benzolring vorzugsweise in 1, 3, 5- Stellung zueinander angeordnet.

In einer bevorzugten Ausführungsform der Erfindung stehen alle übrigen Reste R am Benzolring in Formel (I), welche nicht dem in eckigen Klammern stehenden Rest a entsprechen, jeweils für Wasserstoff.

Das erfindungsgemäße Keton gemäß der allgemeinen Formel (I) ist als Duftspeicherstoff für alle üblichen Duftketone geeignet, insbesondere ausgewählt aus Buccoxim; iso-Jasmon; Methyl-beta-naphthylketon; Moschusindanon; Tonalid/Musk plus; alpha-Damascone, beta-Damascone, delta-Damascone, gamma-Damascone, Damascenon, Damarose, Methyldihydrojasmonat, Menthon, Carvon, Kampfer, Fenchon, alpha-Ionon, beta-Ionon, gamma-Methyl genannt Ionon, Fleuramon, Dihydrojasmon, cis-Jasmon, iso-E-Super®, Methylcedrenylketon oder Methylcedry-Ion, Acetophenon, Methylacetophenon, para-Methoxyacetophenon, Methyl-beta-naphtylkefon, Benzylaceton, Benzophenon, para-Hydroxyphenylbutanon, Sellerie-Keton oder Livescone, 6-Isopropyldecahydro-2-näphton, Dimethyloctenon, Frescomenthe, 4-(1-Ethoxyvinyl)-3,3,5,5-tetramethylcyclohexanon, Methylheptenon, 2-(2-(4-Methyl-3-cyclohexen-1-yl)propyl)cyclopentanon, 1-(p-Menthen-6(2)yl)-1-propanon, 4-(4-Hydro-xy-3-methoxyphenyl)-2-butanon, 2-Acetyl-3,3-dimethylnorbornan, 6,7-Dihydro-1,1,2,3,3-pentamethyl-4(5H)in-danon, 4-Damascol, Dulcinyl oder Cassion, Gelson, Hexalon, Isocyclemon E, Methylcyclocitron, Methyllavendelketon, Orivon, para-tertiärem Butylcyclohexanon, Verdon, Delphon, Muscon, Neobutenon, Plicaton, Velou-ton, 2,4,4,7-Tetramethyl-oct-6-en-3-on, Tetrameran oder Mischungen davon. Bevorzugt können die Ketone ausgewählt sein aus den Damasconen, Carvon, Gamma-Methyl-ionon, Iso-E-Super, 2,4,4,7-Tetramethyl-oct-6-en-3-on, Benzylaceton, Damascenon, Methyldihydrojasmonat, Methyl-cedrylon, Hedion und Gemischen davon. Am meisten bevorzugt sind alle Damascone sowie Damascenone.

Durch Einwirkung von elektromagnetischer Strahlung, insbesondere umfassend die Wellenlängen von 200 bis 400 nm, können die gespeicherten Ketone aus der erfindungsgemäßen Verbindung nach Formel (I) freigesetzt werden.

Gemäß einer besonders bevorzugten Ausführungsform handelt es sich bei dem erfindungsgemäßen Keton gemäß Formel (I) um eine Verbindung, bei dem zwei der Reste R dem genannten Rest a in eckigen Klammern entsprechen, welche zueinander in 1,4-Stellung anbeordnet sind, und wobei es sich jeweils um den gleichen Rest a handelt, und wobei die übrigen vier Reste R für Wasserstoff stehen, und wobei die Reste a vorzugsweise so gewählt sind, dass das erfindungsgemäße Keton bei Einwirkung von Licht, insbesondere umfassend die Wellenlängen von 200 bis 400 nm, eines der zuvor namentlich genannten Duftketone freisetzt, insbesondere Duftketone vom Typ der Damascone.

Gemäß einer bevorzugten Ausführungsform entspricht der Rest a in eckigen Klammern folgendem Rest b: 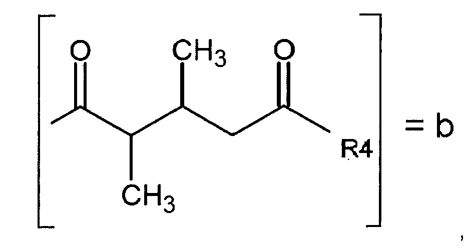 wobei der Rest R4 für einen ggf. substituierten Kohlenwasserstoffrest mit wenigstens 5 C-Atomen steht, der insbesondere einen cyclischen Kohlenwasserstoffrest umfasst.

Besonders bevorzugte Reste b entsprechen den nachfolgenden Resten b1 bis b5:

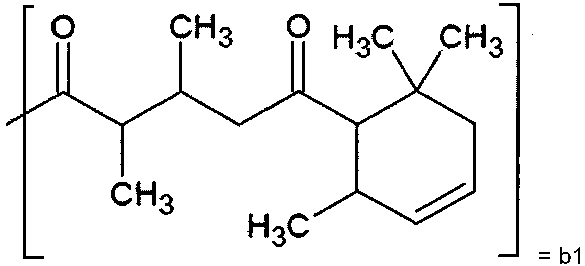

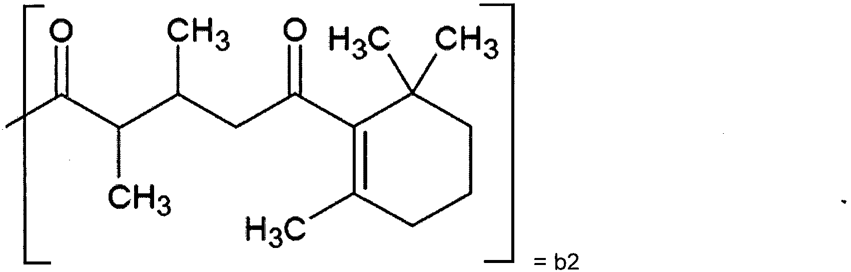

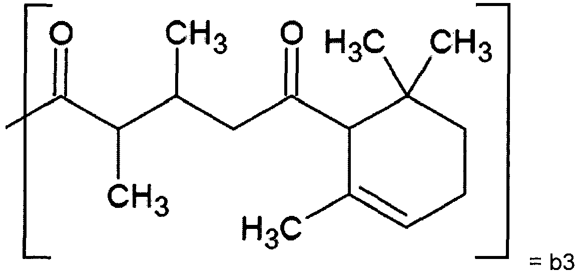

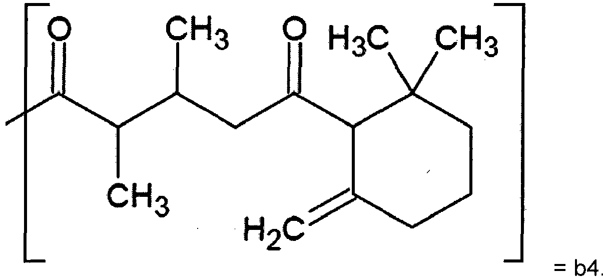

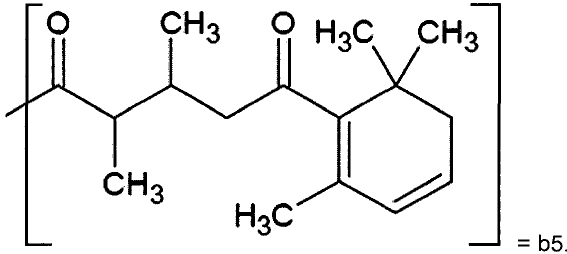

Bevorzugte erfindungsgemäße Ketone gemäß Formel (I) umfassen 2 oder 3 der Reste b1 bis b5, wobei die übrigen Reste R für Wasserstoff stehen. Besonders bevorzugte erfindungsgemäße Ketone gemäß Formel (I) umfassen 2 gleiche Reste, ausgewählt aus b1 bis b5, welche Reste zueinander vorzugsweise in 1,4-Stellung stehen, wobei die übrigen Reste R für Wasserstoff stehen.

Dementsprechend ist ein Beispiel für ein besonders bevorzugtes erfindungsgemäßes Keton das folgende:

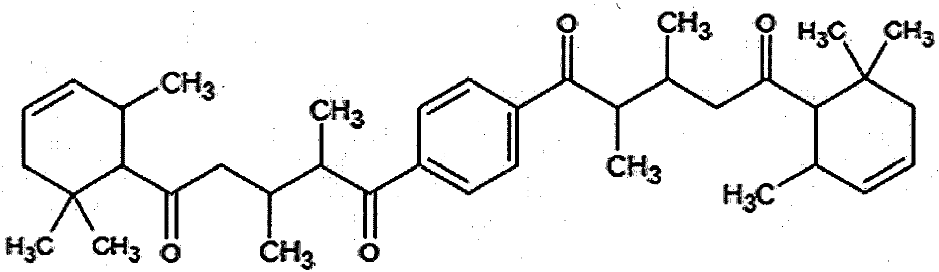

Die erfindungsgemäßen Ketone der vorgenannten Formeln lassen sich sehr stabil in die üblichen Wasch- oder Reinigungsmittelmatrices, in Kosmetika und bestehende Riechstoffkompositionen einarbeiten. Sie ermöglichen eine verzögerte Freisetzung der gespeicherten Duftstoffe, nämlich insbesondere von Damascone in der α-, β-, γ- oder δ-Form sowie von Damascenone, insbesondere β-Damascenone. Diese Ketone verleihen üblichen Wasch- oder Reinigungsmitteln sowie Kosmetika einen besonders lange anhaltenden Frischeeindruck. Insbesondere das getrocknete, gewaschene Textil profitiert von der guten Langzeitfrischeduftwirkung. Die langsame Freisetzung des gespeicherten Riechstoffes erfolgt nach Einwirkung von Licht (elektromagnetische Strahlung), insbesondere umfassend die Wellenlängen von 200 bis 400 nm, wie in der nachfolgender Reaktionsgleichung vereinfacht veranschaulicht:

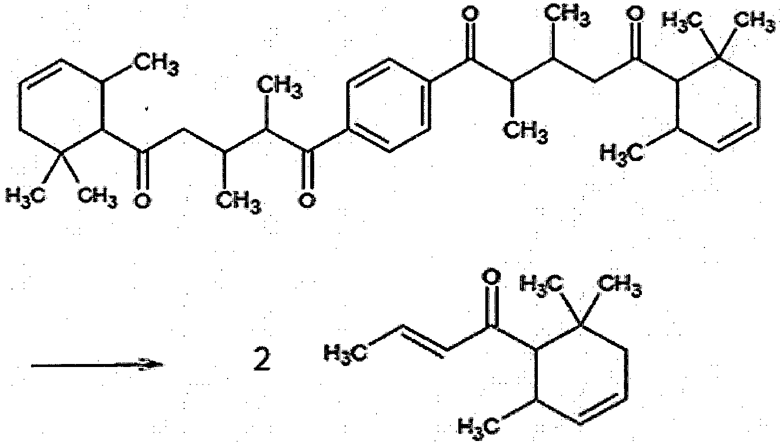

Ein weiterer Gegenstand der vorliegenden Erfindung ist ein Wasch- oder Reinigungsmittel, vorzugsweise ein Waschmittel, Weichspüler oder Wascfihilfsmittel, enthaltend mindestens ein erfindungsgemäßes Keton gemäß Formel (I), wobei das genannte Keton vorzugsweise in Mengen zwischen 0,0001 und 5 Gew.-%, vorteilhafterweise zwischen 0,001 und 4 Gew.-%, weiter vorteilhaft zwischen 0,01 und 3 Gew.-%, insbesondere zwischen 0,1 und 2 Gew.-%, jeweils bezogen auf das gesamte Mittel, enthalten ist. Geeignete Reinigungsmittel sind z.B. Reinigungsmittel für harte Oberflächen, wie vorzugsweise Geschirrspülmittel. Ebenso kann es sich um Reinigungsmittel wie z.B. Haushaltsreiniger, Allzweckreiniger, Fensterreiniger, Fußbodenreiniger usw. handeln. Vorzugsweise kann es sich um ein Produkt zur Reinigung von WC-Becken und Urinalen handeln, vorteilhafterweise um einen Spülreiniger zum Einhängen in das WC-Becken, insbesondere um einen sogenannten WC-Stein.

Gemäß einer bevorzugten Ausführungsform der Erfindung enthält das erfindungsgemäße Wasch- oder Reinigungsmittel mindestens ein Tensid, ausgewählt aus anionischen, kationischen, nichtionischen, zwitterionischen, amphoteren Tensiden oder Mischungen daraus.

Gemäß einer weiteren bevorzugten Ausführungsform der Erfindung liegt das erfindungsgemäße Mittel in fester oder flüssiger Form vor.

Ein weiterer Gegenstand der Erfindung ist ein kosmetisches Mittel, enthaltend mindestens ein Keton gemäß Formel (I), welches das genannte Keton vorzugsweise in Mengen zwischen 0,0001 und 5 Gew.-%, vorteilhafterweise zwischen 0,001 und 4 Gew.-%, weiter vorteilhaft zwischen 0,01 und 3 Gew.-%, insbesondere zwischen 0,1 und 2 Gew.-%, jeweils bezogen auf das gesamte Mittel, enthält.

Ein weiterer Gegenstand der Erfindung ist ein Luftpflegemittel (z.B. Raumlufterfrischer, Raumdeodorant, Raumspray usw.), enthaltend mindestens ein Keton gemäß Formel (I), wobei das genannte Keton vorzugsweise in Mengen zwischen 0,0001 und 50 Gew.-%, vorteilhafterweise zwischen 0,001 und 5 Gew.-%, weiter vorteilhaft zwischen 0,1 und 3 Gew.-%, insbesondere zwischen 0,1 und 2 Gew.-%, jeweils bezogen auf das gesamte Mittel, enthalten ist.

Nach einer weiteren bevorzugten Ausführungsform der Erfindung sind in einem erfindungsgemäßen Mittel (d.h. Wasch- oder Reinigungsmittel, kosmetisches Mittel, oder Luftpflegemittel), insbesondere Wasch- oder Reinigungsmittel, zusätzliche Duftstoffe enthalten, vorzugsweise in Mengen von 0,00001 bis 5 Gew.-%, insbesondere ausgewählt aus der Gruppe umfassend Duftstoffe natürlichen oder synthetischen Ursprungs, bevorzugt leichter flüchtige Duftstoffe, höhersiedende Duftstoffe, feste Duftstoffe und/oder haftfeste Duftstoffe.

Haftfeste Riechstoffe, die im Rahmen der vorliegenden Erfindung mit Vorteil einsetzbar sind, sind beispielsweise etherische Öle wie Angelikawurzelöl, Anisöl, Arnikablütenöl, Basilikumöl, Bayöl, Bergamottöl, Champacablütenöl, Edeltannenöl, Edeltannenzapfenöl, Elemiöl, Eukalyptusöl, Fenchelöl, Fichtennandelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaivabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Neroliöl, Niaouliöl, Olibanumöl, Orangenöl, Origanumöl, Palmarosaöl, Patschuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wiritergrünöl, Ylang-Ylang-Öl, Ysop-Öl, Zimtöl, Zimtblätteröl, Zitronellöl, Zitronenöl sowie Zypressenöl.

Aber auch höhersiedende bzw. feste Riechstoffe natürlichen oder synthetischen Ursprungs können im Rahmen der vorliegenden Erfindung als haftfeste Riechstoffe bzw. Riechstoffgemische, also Duftstoffe eingesetzt werden. Zu diesen Verbindungen zählen die nachfolgend genannten Verbindungen sowie Mischungen aus diesen: Ambrettolid, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylalkohol, Benzylacetat, Benzylbenzoat, Benzylformiat, Benzylvalerianat, Borneol, Bornylacetat, α-Bromstyrol, n-Decylaldehyd, n-Dodecylaldehyd, Eugenof, Eugenolmethylether, Eukalyptol, Farnesol, Fenchon, Fenchylacetat, Geranylacetat, Geranylformiat, Heliotropin, Heptincarbonsäuremethylester, Heptaldehyd, Hy-drochinon- Dimethylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p-Kresol-methylether, Cumarin, p-Methoxyaceto-phenon, Methyl-n-amylketon, Methylanthranilsäure-methylester, p-Methylacetophenon, Methylchavikol, p-Methylchinolin, Methyl-β-naphthylketon, Methyl-n-nonylacetaldehyd, Methyl-n-nonylketon, Muskon, β-Naphtholethylether, β-Naphtholmethylether, Nerol, Nitrobenzol, n-Nonylaldehyd, Nonylakohol, n-Octylaldehyd, p-Oxy-Acetophenon, Pentadekanolid, β-Phenylethylalkohol, Phenylacetaldehyd-Dimethylacetal, Phenylessigsäure, Pulegon, Safrol, Salicylsäureisoamylester, Salicylsäuremethylester, Salicylsäurehexylester, Salicylsäurecyclohexylester, Santalol, Skatol, Terpineol, Thymen, Thymol, γ-Undelacton, Vanilin, Veratrumaldehyd, Zimtaldehyd, Zimatalkohol, Zimtsäure, Zimtsäureethylester, Zimtsäurebenzylester. Zu den leichter flüchtigen Duftstoffen zählen insbesondere die niedriger siedenden Riechstoffe natürlichen oder synthetischen Ursprung, die allein oder in Mischungen eingesetzt werden können. Beispiele für leichter flüchtige Duftstoffe sind Alkylisothiocyanate (Alkylsenföle), Butandion, Limonen, Linalool, Linaylacetat und -Propionat, Menthol, Menthon, Methyl-n-heptenon, Phellandren, Phenylacetaldehyd, Terpinylacetat, Zitral, Zitronellal.

Nach einer weiteren bevorzugten Ausführungsform weist das erfindungsgemäße Mittel (d.h. Wasch- oder Reinigungsmittel, kosmetisches Mittel, oder Luftpflegemittel), insbesondere Wasch- oder Reinigungsmittel, wenigstens eine, vorzugsweise mehrere, aktive Komponenten, insbesondere wasch-, pflege-, reinigungsaktive und/oder kosmetische Komponenten auf, vorteilhafterweise ausgewählt aus der Gruppe umfassend anionische Tenside, kationische Tenside, amphotere Tenside, nichtionische Tenside, Acidifizierungsmittel, Alkalisierungsmittel, Anti-Knitter-Verbindungen, antibakterielle Stoffe, Antioxidantien, Antiredepositionsmittel, Antistatika, Buildersubstanzen, Bleichmittel, Bleichaktivatoren, Bleichstabilisatoren, Bleichkatalysatoren, Bügelhilfsmittel, Cobuilder, Duftstoffe, Einlaufverhinderer, Elektrolyte, Enzyme, Farbschutzstoffe, Färbemittel, Farbstoffe, Farbübertragungsinhibitoren, Fluoreszensmittel, Fungizide, Germizide, geruchskomplexierende Substanzen, Hilfsmittel, Hydrotrope, Klarspüler, Komplexbildner, Konservierungsmittel, Korrosionsinhibitoren, wassermischbare organische Lösungsmittel, optische Aufheller, Parfüme, Parfümträger, Perlglanzgeber, pH-Stellmittel, Phobier- und Imprägniermittel, Polymere, Quell- und Schiebefestmittel, Schauminhibitoren, Schichtsilikate, schmutzab-weisende Stoffe, Silberschutzmittel, Silikonöle, Soilrelease-Wirkstoffe, UV-Schutz-Substanzen, Viskositätsregulatoren, Verdickungsmittel, Verfärbungsinhibitoren, Vergrauungsinhibitoren, Vitamine und/oder Weichspüler. Im Sinne dieser Erfindung beziehen sich Angaben für das erfindungsgemäße Mittel in Gew.-%, wenn nicht anders angegeben, auf das Gesamtgewicht des erfindungsgemäßen Mittels.

Die Mengen der einzelnen Inhaltsstoffe in den erfindungsgemäßen Mitteln (d.h. Waschoder Reinigungsmittel, kosmetisches Mittel oder Luftpflegemittel), insbesondere Waschoder Reinigungsmittel, orientieren sich jeweils am Einsatzzweck der betreffenden Mittel und der Fachmann ist mit den Größenordnungen der einzusetzenden Mengen der Inhaltsstoffe grundsätzlich vertraut oder kann diese der zugehörigen Fachliteratur entnehmen. Je nach Einsatzzweck der erfindungsgemäßen Mittel wird man beispielsweise den Tensidgehalt höher oder niedriger wählen. Üblicherweise kann z. B. der Tensidgehalt beispielsweise von Waschmitteln zwischen 10 und 50 Gew.-%, vorzugsweise zwischen 12,5 und 30 Gew.-% und insbesondere zwischen 15 und 25 Gew.-% betragen, während z.B. Reinigungsmittel für das maschinelle Geschirrspülen z.B. zwischen 0,1 und 10 Gew.-%, vorzugsweise zwischen 0,5 und 7,5 Gew.-% und insbesondere zwischen 1 und 5 Gew.-% Tenside enthalten können.

Die erfindungsgemäßen Mittel (d.h. Wasch- oder Reinigungsmittel, kosmetisches Mittel oder Luftpflegemittel), insbesondere Wasch- oder Reinigungsmittel, können Tenside enthalten, wobei bevorzugt anionische Tenside, nichtionische Tenside und deren Gemische, aber auch kationische Tenside in Frage kommen. Geeignete nichtionische Tenside sind insbesondere Ethoxylierungs- und/oder Propoxylierungsprodukte von Alkylglykosiden und/oder linearen oder verzweigten Alkoholen mit jeweils 12 bis 18 C-Atomen im Alkylteile und 3 bis 20, vorzugsweise 4 bis 10 Alkylethergruppen. Weiterhin sind entsprechende Ethoxylierungs- und/oder Propoxylierungsprodukte von N-Alkylaminen, vicinalen Diolen, Fettsäureestern und Fettsäureamiden, die hinsichtlich des Alkylteils den genannten langkettigen Alkoholderivaten entsprechen, sowie von Alkylphenolen mit 5 bis 12 C-Atomen im Alkylrest brauchbar.

Geeignete anionische Tenside sind insbesondere Seifen und solche, die Sulfat- oder Sulfonat-Gruppen mit bevorzugt Alkaliionen als Kationen enthalten. Verwendbare Seifen sind bevorzugt die Alkalisalze der gesättigten oder ungesättigten Fettsäuren mit 12 bis 18 C-Atomen. Derartige Fettsäuren können auch in nicht vollständig neutralisierter Form eingesetzt werden. Zu den brauchbaren Tensiden des Sulfat-Typs gehören die Salze der Schwefelsäurehalbester von Fettalkoholen mit 12 bis 18 C-Atomen und die Sulfatierungsprodukte der genannten nichtionischen Tenside mit niedrigen Ethoxylierungsgrad. Zu den verwendbaren Tensiden vom Sulfonat-Typ gehören lineare Alkylbenzolsulfonate mit 9 bis 14 C-Atomen im Alkylteil, Alkansulfonate mit 12 bis 18 C-Atomen, sowie Olefinsulfonate mit 12 bis 18 C-Atomen, die bei der Umsetzung entsprechender Monoolefine mit Schwefeltrioxid entstehen, sowie alpha-Sulfofettsäureester, die bei der Sulfonierung von Fettsäuremethyl- oder -ethylestern entstehen.

Kationische Tenside werden vorzugsweise unter den Esterquats und/oder den quaternären Ammoniumverbindungen (QAV) gemäß der allgemeinen Formel (R^{I})(R^{II})(R^{III})(R^{IV})N⁺ X- ausgewählt, in der R^{I} bis R^{IV} für gleiche oder verschiedene C₁₋₂₂-Alkylreste, C₇₋₂₈-Arylalkylreste oder heterozyklische Reste stehen, wobei zwei oder im Falle einer aromatischen Einbindung wie im Pyridin sogar drei Reste gemeinsam mit dem Stickstoffatom den Heterozyklus, z.B. eine Pyridinium- oder Imidazoliniumverbindung, bilden, und X-für Halogenidionen, Sulfationen, Hydroxidionen oder ähnliche Anionen steht. QAV sind durch Umsetzung tertiärer Amine mit Alkylierungsmitteln, wie z.B. Methylchlorid, Benzylchlorid, Dimethylsulfat, Dodecylbromid, aber auch Ethylenoxid herstellbar. Die Alkylierung von tertiären Aminen mit einem langen Alkyl-Rest und zwei Methyl-Gruppen gelingt besonders leicht, auch die Quaternierung von tertiären Aminen mit zwei langen Resten und einer Methyl-Gruppe kann mit Hilfe von Methylchlorid unter milden Bedingungen durchgeführt werden. Amine, die über drei lange Alkyl-Reste oder Hydroxy-substituierte Alkyl-Reste verfügen, sind wenig reaktiv und werden z.B. mit Dimethylsulfat quaterniert. In Frage kommende QAV sind beispielweise Benzalkoniumchlorid (N Alkyl-N,N dimethyl-benzylammonium-chlorid), Benzalkon B (m,p-Dichlorbenzyldimethyl-C₁₂-alkylammoniumchlorid, Benzoxoniumchlorid (Benzyl-dodecyl-bis-(2-hydroxyethyl)-ammoniumchlorid), Cetrimoniumbromid (N-Hexadecyl-N,N-trimethyl-ammoniumbromid), Benzetoniumchlorid (N,N Dimethyl-N [2-[2-[p-(1,1,3,3-tetramethylbutyl)phenoxy]-ethoxy]-ethyl]-benzylammoniumchlorid), Dialkyldimethylammoniumchloride wie Di-n-decyl-dimethyl-ammoniumchlorid, Didecyldimethylammonium-bromid, Dioctyl-dimethyl-ammoniumchlorid, 1-Cetylpyridiniumchlorid und Thiazolinjodid sowie deren Mischungen. Bevorzugte QAV sind die Benzalkoniumchloride mit C₈-C₂₂-Alkylresten, insbesondere C₁₂-C₁₄-Alkyl-benzyl-dimethylammoniumchlorid.

Bevorzugte Esterquats sind Methyl-N-(2-hydroxyethyl)-N,N-di(talgacyl-oxyethyl)ammonium-methosulfat, Bis-(palmitoyl)-ethyl-hydroxyethyl-methyl-ammonium-methosulfat oder Methyl-N,N-bis(acyloxyethyl)-N-(2-hydroxyethyl)ammonium-methosulfat.

Tenside sind in den erfindungsgemäßen Mitteln (d.h. Wasch- oder Reinigungsmittel, kosmetisches Mittel, oder Luftpflegemittel), insbesondere Wasch- oder Reinigungsmitteln, in Mengenanteilen von vorzugsweise 5 Gew.-% bis 50 Gew.-%, insbesondere von 8 Gew.-% bis 30 Gew.-%, enthalten. Insbesondere in Wäschenachbehandlungsmitteln werden vorzugsweise bis zu 30 Gew.-%, insbesondere 5 Gew.-% bis 15 Gew.-% Tenside, unter diesen bevorzugt wenigstens anteilsweise Kationtenside, eingesetzt.

Ein erfindungsgemäßes Mittel, insbesondere Wasch- oder Reinigungsmittel, enthält vorzugsweise mindestens einen wasserlöslichen und/oder wasserunlöslichen, organischen und/oder anorganischen Builder. Zu den wasserlösliche organischen Buildersubstanzen gehören Polycarbonsäuren, insbesondere Citronensäure und Zuckersäuren, monomere und polymere Aminopolycarbonsäuren, insbesondere Methylglycindiessigsäure, Nitrilotriessigsäure und Ethylendiamintetraessigsäure sowie Polyasparaginsäure, Polyphosphonsäuren, insbesondere Aminotris(methylenphosphonsäure), Ethylendiamintetrakis(methylenphosphonsäure) und 1-Hydroxyethan-1,1-diphosphonsäure, polymere Hydroxyverbindungen wie Dextrin sowie polymere (Poly-)carbonsäuren, polymere Acrylsäuren, Methacrylsäuren, Maleinsäuren und Mischpolymere aus diesen, die auch geringe Anteile polymerisierbarer Substanzen ohne Carbonsäurefunktionalität einpolymerisiert enthalten können. Die organischen Buildersubstanzen können, insbesondere zur Herstellung flüssiger Mittel, in Form wäßriger Lösungen, vorzugsweise in Form 30- bis 50-gewichtsprozentiger wäßriger Lösungen eingesetzt werden. Alle genannten Säuren werden in der Regel in Form ihrer wasserlöslichen Salze, insbesondere ihre Alkalisalze, eingesetzt.

Organische Buildersubstanzen können gewünschtenfalls in Mengen bis zu 40 Gew.-%, insbesondere bis zu 25 Gew.-% und vorzugsweise von 1 Gew.-% bis 8 Gew.-% enthalten sein. Mengen nahe der genannten Obergrenze werden vorzugsweise in pastenförmigen oder flüssigen, insbesondere wasserhaltigen, erfindungsgemäßen Mitteln eingesetzt. Erfindungsgemäße Wäschenachbehandlungsmittel, wie z.B. Weichspüler, können gegebenenfalls auch frei von organischem Builder sein.

Als wasserlösliche anorganische Buildermaterialien kommen insbesondere Alkalisilikate und Polyphosphate, vorzugsweise Natriumtriphosphat, in Betracht. Als wasserunlösliche, wasserdispergierbare anorganische Buildermaterialien können insbesondere kristalline oder amorphe Alkalialumosilikate, gewünschtenfalls in Mengen von bis zu 50 Gew.-%, vorzugsweise nicht über 40 Gew.-% und in flüssigen Mitteln insbesondere von 1 Gew.-% bis 5 Gew.-%, eingesetzt werden. Unter diesen sind die kristallinen Natriumalumosilikate in Waschmittelqualität, insbesondere Zeolith A, P und gegebenenfalls X, bevorzugt. Mengen nahe der genannten Obergrenze werden vorzugsweise in festen, teilchenförmigen Mitteln eingesetzt. Geeignete Alumosilikate weisen insbesondere keine Teilchen mit einer Korngröße über 30 µm auf und bestehen vorzugsweise zu wenigstens 80 Gew.-% aus Teilchen mit einer Größe unter 10 µm. Es ist aber besonders bevorzugt, auf wasserunlösliche Buildermaterialien zumindest weitestgehend zu verzichten, so dass sie, wenn überhaupt, vorzugsweise nur in geringen Mengen eingesetzt werden, z.B. in Mengen < 5 Gew.-% oder < 1 Gew.-%, bezogen auf das gesamte Mittel.

Geeignete Substitute beziehungsweise Teilsubstitute für das genannte Alumosilikat sind kristalline Alkalisilikate, die allein oder im Gemisch mit amorphen Silikaten vorliegen können. Die in den erfindungsgemäßen Mitteln als Gerüststoffe brauchbaren Alkalisilikate weisen vorzugsweise ein molares Verhältnis von Alkalioxid zu SiO₂ unter 0,95, insbesondere von 1:1,1 bis 1:12 auf und können amorph oder kristallin vorliegen. Bevorzugte Alkalisilikate sind die Natriumsilikate, insbesondere die amorphen Natriumsilikate, mit einem molaren Verhältnis Na₂O:SiO₂ von 1:2 bis 1:2,8. Als kristalline Silikate, die allein oder im Gemisch mit amorphen Silikaten vorliegen können, werden vorzugsweise kristalline Schichtsilikate der allgemeinen Formel Na₂SiₓO₂ₓ₊₁ y H₂O eingesetzt, in der x, das sogenannte Modul, eine Zahl von 1,9 bis 4 und y eine Zahl von 0 bis 20 ist und bevorzugte Werte für x 2, 3 oder 4 sind. Bevorzugte kristalline Schichtsilikate sind solche, bei denen x in der genannten allgemeinen Formel die Werte 2 oder 3 annimmt. Insbesondere sind sowohl ß- als auch δ-Natriumdisilikate (Na₂Si₂O5 y H₂O) bevorzugt. Auch aus amorphen Alkalisilikaten hergestellte, praktisch wasserfreie kristalline Alkalisilikate der obengenannten allgemeinen Formel, in der x eine Zahl von 1,9 bis 2,1 bedeutet, können in erfindungsgemäßen Mitteln eingesetzt werden. In einer weiteren bevorzugten Ausführungsform erfindungsgemäßer Mittel wird ein kristallines Natriumschichtsilikat mit einem Modul von 2 bis 3 eingesetzt, wie es aus Sand und Soda hergestellt werden kann. Kristalline Natriumsilikate mit einem Modul im Bereich von 1,9 bis 3,5 werden in einer weiteren bevorzugten Ausführungsform erfindungsgemäßer Mittel eingesetzt. Falls als zusätzliche Buildersubstanz auch Alkalialumosilikat, insbesondere Zeolith, vorhanden ist, beträgt das Gewichtsverhältnis Alumosilikat zu Silikat, jeweils bezogen auf wasserfreie Aktivsubstanzen, vorzugsweise 1:10 bis 10:1. In Mitteln, die sowohl amorphe als auch kristalline Alkalisilikate enthalten, beträgt das Gewichtsverhältnis von amorphem Alkalisilikat zu kristallinem Alkalisilikat vorzugsweise 1:2 bis 2:1 und insbesondere 1:1 bis 2:1.

Buildersubstanzen sind gewünschtenfalls in den erfindungsgemäßen Mitteln, insbesondere Wasch- oder Reinigungsmittel, vorzugsweise in Mengen bis zu 60 Gew.-%, insbesondere von 5 Gew.-% bis 40 Gew.-%, enthalten. Erfindungsgemäße Wäschenachbehandlungsmittel, wie z.B. Weichspüler, sind vorzugsweise frei von anorganischem Builder. Als geeignete Persauerstoffverbindungen kommen insbesondere organische Persäüren beziehungsweise persaure Salze organischer Säuren, wie Phthalimidopercapronsäure, Perbenzoesäure oder Salze der Diperdodecandisäure, Wasserstoffperoxid und unter den Anwendüngsbedingungen Wasserstoffperoxid abgebende anorganische Salze, wie Perborat, Percarbonat und/oder Persilikat, in Betracht. Sofern feste Persauerstoffverbindungen eingesetzt werden sollen, können diese in Form von Pulvern oder Granulaten verwendet werden, die auch in im Prinzip bekannter Weise umhüllt sein können. Besonders bevorzugt wird Alkalipercarbonat, Alkaliperborat-Monohydrat oder insbesondere in flüssigen Mitteln Wasserstoffperoxid in Form wäßriger Lösungen, die 3 Gew.-% bis 10 Gew.-% Wasserstoffperoxid enthalten, ggf. eingesetzt. Falls ein erfindungsgemäßes Mittel Bleichmittel, wie vorzugsweise Persauerstoffverbindungen, enthält, sind diese in Mengen von vorzugsweise bis zu 50 Gew.-%, insbesondere von 5 Gew.-% bis 30 Gew.-%, vorhanden. Der Zusatz geringer Mengen bekannter Bleichmittelstabilisatoren wie beispielsweise von Phosphonaten, Boraten bzw. Metaboraten und Metasilikaten sowie Magnesiumsalzen wie Magnesiumsulfat kann zweckdienlich sein.

Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atom-zahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat, 2,5-Diacetoxy-2,5-dihydrofuran und Enolester sowie acetyliertes Sorbitol und Mannitol beziehungsweise deren Mischungen (SORMAN), acylierte Zuckerderivate, insbesondere Pentaacetylglukose (PAG), Pentaacetylfruktose, Tetraacetylxylose und Octaacetyllactose sowie acetyliertes, gegebenenfalls N-alkyliertes Glucamin und Glu-conolacton, und/oder N-acylierte Lactame, beispielsweise N-Benzoylcaprolactam.

Hydrophil substituierte Acylacetale und Acyllactame werden ebenfalls bevorzugt eingesetzt. Auch Kombinationen konventioneller Bleichaktivatoren können eingesetzt werden. Derartige Bleichaktivatoren können im üblichen Mengenbereich, vorzugsweise in Mengen von 1 Gew.-% bis 10 Gew.-%, insbesondere 2 Gew.-% bis 8 Gew.-%, bezogen auf gesamtes Mittel, enthalten sein.

Zusätzlich zu den oben aufgeführten konventionellen Bleichaktivatoren oder an deren Stelle können auch Sulfonimine und/oder bleichverstärkende Übergangsmetallsalze beziehungsweise Übergangsmetallkomplexe als sogenannte Bleichkatalysatoren enthalten sein.

Als in den Mitteln verwendbare Enzyme kommen solche aus der Klasse der Proteasen, Cutinasen, Amylasen, Pullulanasen, Hemicellulasen, Cellulasen, Lipasen, Oxidasen und Peroxidasen sowie deren Gemische in Frage. Besonders geeignet sind aus Pilzen oder Bakterien, wie Bacillus subtilis, Bacillus licheniformis, Streptomyces griseus, Humicola lanuginosa, Humicola insolens, Pseudomonas pseudoalcaligenes oder Pseudomonas cepacia gewonnene enzymatische Wirkstoffe. Die gegebenenfalls verwendeten Enzyme können an Trägerstoffen adsorbiert und/oder in Hüllsubstanzen eingebettet sein, um sie gegen vorzeitige Inaktivierung zu schützen. Sie sind gewünschtenfalls in den erfindungsgemäßen Mitteln vorzugsweise in Mengen nicht über 5 Gew.-%, insbesondere von 0,2 Gew.-% bis 2 Gew.-%, enthalten.

Die Mittel können ggf. als optische Aufheller beispielsweise Derivate der Diaminostilbendisulfonsäure beziehungsweise deren Alkalimetallsalze enthalten. Geeignet sind zum Beispiel Salze der 4,4'-Bis(2-anilino-4-morpholino-1,3,5-triazinyl-6-amino)stilben-2,2'-di-sulfonsäure oder gleichartig aufgebaute Verbindungen, die anstelle der Morpholino-Gruppe eine Diethanolaminogruppe, eine Methylaminogruppe, eine Anilinogruppe oder eine 2-Methoxyethylaminogruppe tragen.

Zu den geeigneten Schauminhibitoren gehören beispielsweise Organopolysiloxane und deren Gemische mit mikrofeiner, gegebenenfalls silanierter Kieselsäure sowie Paraffinwachse und deren Gemische mit silanierter Kieselsäure oder Bisfettsäure-alkylendiamiden. Mit Vorteilen werden auch Gemische aus verschiedenen Schauminhibitoren verwendet, zum Beispiel solche aus Silikonen, Paraffinen oder Wachsen. Vorzugsweise sind die Schauminhibitoren, insbesondere Silikon- und/oder Paraffin-haltige Schaurriinhibiforen, an eine granulare, in Wasser lösliche beziehungsweise dispergierbare Trägersubstanz gebunden. Insbesondere sind dabei Mischungen aus Paraffinwachsen und Bistearylethylendiamiden bevorzugt.

Zusätzlich können die Mittel auch Komponenten enthalten, welche die Öl- und Fettauswaschbarkeit aus Textilien positiv beeinflussen, sogenannte soil release-Wirkstoffe. Dieser Effekt wird besonders deutlich, wenn ein Textil verschmutzt wird, das bereits vorher mehrfach mit einem erfindungsgemäßen Mittel, das diese öl- und fettlösende Komponente enthält, gewaschen wurde. Zu den bevorzugten öl- und fettlösenden Komponenten zählen beispielsweise nicht-ionische Celluloseether wie Methylcellulose und Methylhydroxypropy-Icellulose mit einem Anteil an Methoxyl-Gruppen von 15 bis 30 Gew.-% und an Hydroxypropoxyl-Gruppen von 1 bis 15 Gew.-%, jeweils bezogen auf den nichtionischen Cellulose-ether, sowie die aus dem Stand der Technik bekannten Polymere der Phthalsäure und/oder der Terephthalsäure bzw. von deren Derivaten mit monomeren und/oder polymeren Diolen, insbesondere Polymere aus Ethylenterephthalaten und/oder Polyethylenglykolterephthalaten oder anionisch und/oder nichtionisch modifizierten Derivaten von diesen.

Die Mittel können auch Farbübertragungsinhibitoren, vorzugsweise in Mengen von 0,1 Gew.-% bis 2 Gew.-%, insbesondere 0,1 Gew.-% bis 1 Gew.-%, enthalten, die in einer bevorzugten Ausgestaltung der Erfindung Polymere aus Vinylpyrrolidon, Vinylimidazol, Vi-nylpyridin-N-Oxid oder Copolymere aus diesen sind.

Vergrauungsinhibitoren haben die Aufgabe, den von der Textilfaser abgelösten Schmutz in der Flotte suspendiert zu halten. Hierzu sind wasserlösliche Kolloide meist organischer Natur geeignet, beispielsweise Stärke, Leim, Gelatine, Salze von Ethercarbonsäuren oder Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich andere als die obengenannten Stärkederivate verwenden, zum Beispiel Aldehydstärken. Bevorzugt können Celluloseether, wie Carboxymethylcellulose (Na-Salz), Methylcellulose, Hydroxyalkylcellulose und Mischether, wie Methylhydroxyethylcellulose, Methylhydroxypropylcellulose, Methylcarboxymethylcellulose und deren Gemische, beispielsweise in Mengen von 0,1 bis 5 Gew.-%, bezogen auf die Mittel, eingesetzt werden.

Zu den in den erfindungsgemäßen Mitteln, insbesondere wenn sie in flüssiger oder pastöser Form vorliegen, verwendbaren organischen Lösungsmitteln gehören Alkohole mit 1 bis 4 C-Atomen, insbesondere Methanol, Ethanol, Isopropanol und tert.-Butanol, Diole mit 2 bis 4 C-Atomen, insbesondere Ethylenglykol und Propylenglykol, sowie deren Gemische und die aus den genannten Verbindungsklassen ableitbaren Ether. Derartige wassermischbare Lösungsmittel sind in den erfindungsgemäßen Mitteln vorzugsweise in Mengen von nicht über 30 Gew.-%, insbesondere von 6 Gew.-% bis 20 Gew.-%, vorhanden.

Zur Einstellung eines gewünschten, sich durch die Mischung der übrigen Komponenten nicht von selbst ergebenden pH-Werts können die erfindungsgemäßen Mittel system- und umweltverträgliche Säuren, insbesondere Citronensäure, Essigsäure, Weinsäure, Äpfelsäure, Milchsäure, Glykolsäure, Bernsteinsäure, Glutarsäure und/oder Adipinsäure, aber auch Mineralsäuren, insbesondere Schwefelsäure, oder Basen, insbesondere Ammonium- oder Alkalihydroxide, enthalten. Derartige pH-Regulatoren sind optional in den erfindungsgemäßen Mitteln vorzugsweise nicht über 20 Gew.-%, insbesondere von 1,2 Gew.-% bis 17 Gew.-%, enthalten.

Die Herstellung fester erfindungsgemäßer Mittel (d.h. insbesondere Wasch- oder Reinigungsmittel) bereitet keine Schwierigkeiten und kann im Prinzip bekannter Weise, zum Beispiel durch Sprühtrocknen oder Granulation, erfolgen, wobei optionale Persauerstoffverbindung und optionaler Bleichkatalysator gegebenenfalls später zugesetzt werden. Zur Herstellung erfindungsgemäßer Mittel mit erhöhtem Schüttgewicht, insbesondere im Bereich von 650 g/l bis 950 g/l, ist ein einen Extrusionsschritt aufweisendes Verfahren bevorzugt. Die Herstellung flüssiger erfindungsgemäßer Mittel bereitet ebenfalls keine Schwierigkeiten und kann ebenfalls in bekannter Weise erfolgen.

Die Herstellung der erfindungsgemäßen Ketone wird im Beispielteil exemplarisch anhand der Herstellung eines δ-Damascone-enthaltenden Duftspeicherstoffes beschrieben. Über diese prinzipielle Syntheseroute sind auch die anderen Ketone der allgemeinen Formel (I) zugänglich.

Gemäß einer bevorzugten Ausführungsform kann die erfindungsgemäße Lehre dazu eingesetzt werden, den Parfümanteil in Wasch-, Reinigungs- und Körperpflegemitteln signifikant herabzusetzen. Dadurch ist es möglich, parfümierte Produkte auch für solche besonders empfindlichen Konsumenten anzubieten, die normal parfümierte Produkte aufgrund spezieller Unverträglichkeiten und Irritationen nur eingeschränkt oder gar nicht verwenden können.

Ein bevorzugtes erfindungsgemäßes festes, insbesondere pulverförmiges Waschmittel kann neben dem erfindungsgemäßen Keton insbesondere noch Komponenten enthalten, die z.B. ausgewählt sind aus den folgenden:
- Aniontenside, wie vorzugsweise Alkylbenzolsulfonat, Alkylsulfat, z.B. in Mengen von vorzugsweise 5-30 Gew.-%
- Nichtionische Tenside, wie vorzugsweise Fettalkoholpolyglycolether, Alkylpolyglucosid, Fettsäureglucamid z.B. in Mengen von vorzugsweise 0,5-15 Gew.-%
- Gerüststoffe, wie z.B. Zeolith, Polycarboxylat, Natriumcitrat, in Mengen von z.B. 0-70 Gew.-%, vorteilhafterweise 5-60 Gew.-%, vorzugsweise 10-55 Gew.-%, insbesondere 15-40 Gew.-%,
- Alkalien, wie z.B. Natriumcarbonat, in Mengen von z.B. 0-35 Gew.-% vorteilhafterweise 1-30 Gew.-%, vorzugsweise 2-25 Gew.-%, insbesondere 5-20 Gew.-%,
- Bleichmittel, wie z.B. Natriumperborat, Natriumpercarbonat, in Mengen von z.B. 0-30 Gew.-% vorteilhafterweise 5-25 Gew.-%, vorzugsweise 10-20 Gew.-%,
- Korrosionsinhibitoren, z.B. Natriumsilicat, in Mengen von z.B. 0-10 Gew.-%, vorteilhafterweise 1-6 Gew.-%, vorzugsweise 2-5 Gew.-%, insbesondere 3-4 Gew.-%,
- Stabilisatoren, z.B. Phosphonate, vorteilhafterweise 0-1 Gew.-%,
- Schauminhibitor, z.B. Seife, Siliconöle, Paraffine vorteilhafterweise 0-4 Gew.-%, vorzugsweise 0,1-3 Gew.-%, insbesondere 0,2-1 Gew.-%,
- Enzyme, z.B. Proteasen, Amylasen, Cellulasen, Lipasen, vorteilhafterweise 0-2 Gew.-%, vorzugsweise 0,2-1 Gew.-%, insbesondere 0,3-0,8 Gew.-%,
- Vergrauungsinhibitor, z.B. Carboxymethylcellulose, vorteilhafterweise 0-1 Gew.-%,
- Verfärbungsinhibifor, z.B. Polyvinylpyrrolidon-Derivate, vorzugsweise 0-2 Gew.-%,
- Stellmittel, z.B. Natriumsulfat, vorteilhafterweise 0-20 Gew.-%,
- Optische Aufheller, z.B. Stilben-Derivat, Biphenyl-Derivat, vorteilhafterweise 0-0,4 Gew.-%, insbesondere 0,1-0,3 Gew.-%,
- ggf. weitere Duftstoffe
- ggf. Wasser
- ggf. Seife
- ggf. Bleichaktivatoren
- ggf. Cellulosderivate
- ggf. Schmutzabweiser,

Gew.-% jeweils bezogen auf das gesamte Mittel.

In einer anderen bevorzugten Ausführungsform der Erfindung liegt das Mittel in flüssiger Form vor, vorzugsweise in Gelform. Bevorzugte flüssige Wasch- oder Reinigungsmittel sowie Kosmetika haben Wassergehalte von z.B. 10-95 Gew.-%, vorzugsweise 20-80 Gew.-% und insbesondere 30-70 Gew.-%, bezogen auf das gesamte Mittel. Im Falle von flüssigen Konzentraten kann der Wassergehalt auch besonders gering sein, z.B. < 30 Gew.-%, vorzugsweise < 20 Gew.-%, insbesondere < 15 Gew.-% betragen, Gew.-% jeweils bezogen auf das gesamte Mittel. Die flüssigen Mittel können auch nichtwässrige Lösungsmittel enthalten.

Ein bevorzugtes erfindungsgemäßes flüssiges, insbesondere gelförmiges Waschmittel kann neben dem erfindungsgemäßen Keton insbesondere noch Komponenten enthalten, die z.B. ausgewählt sind aus den folgenden:
- Aniontenside, wie vorzugsweise Alkylbenzolsulfonat, Alkylsulfat, z.B. in Mengen von vorzugsweise 5-40 Gew.-%
- Nichtionische Tenside, wie vorzugsweise Fettalkoholpolyglycolether, Alkylpolyglucosid, Fettsäureglucamid z.B. in Mengen von vorzugsweise 0,5-25 Gew.-%
- Gerüststoffe, wie z.B. Zeolith, Polycarboxylat, Natriumcitrat, vorteilhafterweise 0-15 Gew.-%, vorzugsweise 0,01-10 Gew.-%, insbesondere 0,1-5 Gew.-%,
- Schauminhibitor, z.B. Seife, Siliconöle, Paraffine, in Mengen von z.B. 0-10 Gew.-%, vorteilhafterweise 0,1-4 Gew.-%, vorzugsweise 0,2-2 Gew.-%, insbesondere 1-3 Gew.-%,
- Enzyme, z.B. Proteasen, Amylasen, Cellulasen, Lipasen, in Mengen von z.B. 0-3 Gew.-%, vorteilhafterweise 0,1-2 Gew.-%, vorzugsweise 0,2-1 Gew.-%, insbesondere 0,3-0,8 Gew.-%,
- Optische Aufheller, z.B. Stilben-Derivat, Biphenyl-Derivat, in Mengen von z.B. 0-1 Gew.-%, vorteilhafterweise 0,1-0,3 Gew.-%, insbesondere 0,1-0,4 Gew.-%,
- ggf. weitere Duftstoffe
- ggf. Stabilisatoren,
- Wasser
- ggf. Seife, in Mengen von z.B. 0-25 Gew.-%, vorteilhafterweise 1-20 Gew.-%, vorzugsweise 2-15 Gew.-%, insbesondere 5-10 Gew.-%,
- ggf. Lösungsmittel (vorzugsweise Alkohole), vorteilhafterweise 0-25 Gew.-%, vorzugsweise 1-20 Gew.-%, insbesondere 2-15 Gew.-%, Gew.-% jeweils bezogen auf das gesamte Mittel.

Ein bevorzugter erfindungsgemäßer flüssiger Weichspüler kann neben dem erfindungsgemäßen Keton insbesondere noch Komponenten enthalten, die ausgewählt sind aus den folgenden:
- Kationische Tenside, wie insbesondere Esterquats, z.B. in Mengen von 5-30 Gew.-%,
- Cotenside, wie z.B. Glycerolmonostearat, Stearinsäure, Fettalkohole, Fettalkoholethoxylate, z.B. in Mengen von 0-5 Gew.-%, vorzugsweise 0,1-4 Gew.-%,
- Emulgatoren, wie z.B. Fettaminethoxylate, z.B. in Mengen von 0-4 Gew.-%, vorzugsweise 0,1-3 Gew.-%,
- ggf. weitere Duftstoffe
- Farbstoffe, vorzugsweise im ppm-Bereich
- Stabilisatoren, vorzugsweise im ppm-Bereich
- Lösemittel, wie z.B. Wasser, in Mengen von vorzugsweise 60-90 Gew.-%,
- Gew.-% jeweils bezogen auf das gesamte Mittel.

Ein weiterer Gegenstand der Erfindung ist ein Verfahren zur lang anhaltenden Beduftung von Oberflächen, wobei ein Keton gemäß der Formel (I) oder ein erfindungsgemäßes Wasch- oder Reinigungsmittel auf die zu beduftende Oberfläche (z.B. Textil, Geschirr, Fußboden) aufgebracht wird und die genannte Oberfläche anschließend einer elektromagnetischen Strahlung, insbesondere umfassend die Wellenlängen von 200 bis 400 nm, ausgesetzt wird.

Ein weiterer Gegenstand der Erfindung ist ein Verfahren zur lang anhaltenden Raumbeduftung, wobei ein erfindungsgemäßes Luftpflegemittel einer elektromagnetischen Strahlung, insbesondere umfassend die Wellenlängen von 200 bis 400 nm, ausgesetzt wird.

### Beispiel:

### Darstellung eines Ketons der allgemeinen Formel (I):

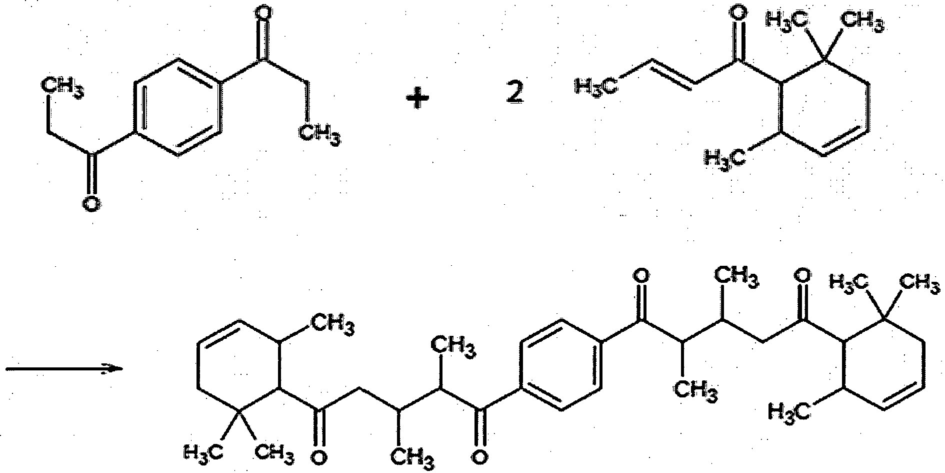

Das Di-Lithiumenolat von 0,43 g (2,3 mmol) 1,1'-Benzyl-1.4-diyldipropan-1-on wurde durch Umsetzung in 10 ml wasserfreiem Tetrahydrofuran mit 5,1 mmol LDA (aus 5,1 mmol Diisopropylamin und 6 mmol einer 1.6 M n-BuLi-Lösung in n-Hexan, 1 Stunde Rühren bei -78 °C in 15 ml THF) durch Zutropfen bei -78 °C über einen Zeitraum von 1 Stunde hergestellt. Das Bis-Enolat wurde dann bei gleicher Temperatur mit 5,52 mmol Cer(III)chlorid (2,05 g, im Vakuum getrocknet) in 15 ml THF versetzt und 30 min bei-78 °C gerührt. Anschließend wurde innerhalb von 30 min unter Rührers 0,96 g Damascon zugetropft und der Ansatz innerhalb von 5 Stunden auf Raumtemperatur erwärmt. Die Reaktionslösung wurde mit 40 ml gesättigter wässriger Ammoniumchloridlösung versetzt und zweimal mit 50 ml Ether extrahiert. Die organische Phase wurde mit Wasser, gesättigter NaCI-Lösung gewaschen und über MgSO₄ getrocknet. Das nach Abziehen des Lösungsmittels verbleibende Rohmaterial wurde durch Wäschen mit Pentan gereinigt. Es wurde ein farbloses Öl erhalten, das weiter säulenchomatographisch (Fließmittel Petrolether:Ethylacetat=95:5) gereinigt wurde. Es wurden das monosubstituierte und das disubstituierte Produkt als Gemisch im Verhältniss 1:5 in Form eines farblosen Öls erhalten. Das auf diese Weise hergestellte disubstituierte Produkt zeigte bei der Anwendung in Waschmitteln und Weichspülern bei der Textilbehandlung eine sehr gute Duftwirkung. Insbesondere wurde eine bessere Dauerhaftigkeit des Dufteindruckes auf der damit gewaschenen und danach getrockneten Wäsche gefunden, verglichen mit Waschmitteln und Weichspülern die eine äquivalente Menge δ-Damascone enthielten, ansonsten aber gleichgestaltet waren. Der frische Dufteindruck der Textilien hielt deutlich länger vor, sowohl nach Leinentrocknung wie insbesondere nach Trocknung im Maschinentrockner.

## Patentansprüche

1. Verbindung der allgemeinen Formel (I), 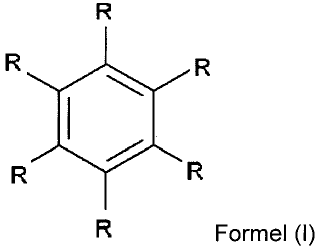 wobei zumindest zwei der Reste R in Formel (I) für den in eckigen Klammern gezeichneten Rest a stehen, 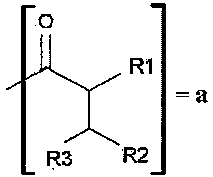
und wobei die übrigen Reste R in Formel (I), unabhängig voneinander, jeweils für Wasserstoff, ein Halogenatom, -NO₂, eine lineare oder verzweigte, substituierte oder unsubstituierte Alkoxygruppe mit 1 bis 15 C-Atomen, eine lineare oder verzweigte, substituierte oder unsubstituierte Alkylgruppe mit 1 bis 15 C-Atomen, einen Arylrest, einen Cycloalkylrest, Acylrest, -OH, -NH-Alkyl, -NH₂ oder -N(Alkyl)₂ stehen,
und wobei für jeden in eckigen Klammern stehenden Rest a, unabhängig voneinander gilt, dass
R2 für einen substituierten Kohlenwasserstoffrest steht, der mindestens eine C=O-Gruppe aufweist,
R1 für eine lineare oder verzweigte, substituierte oder unsubstituierte Alkylgruppe mit 1 bis 6 C-Atomen, insbesondere für eine Methylgruppe steht, und
R3 für Wasserstoff, ein Halogenatom, -NO₂, eine lineare oder verzweigte, substituierte oder unsubstituierte Alkoxygruppe mit 1 bis 15 C-Atomen, eine lineare oder verzweigte, substituierte oder unsubstituierte Alkylgruppe mit 1 bis 15 C-Atomen, einen Arylrest, einen Cycloalkylrest, Acylrest, -OH, -NH-Alkyl, -NH₂ oder -N(Alkyl)₂ steht.

2. Keton nach Anspruch 1, **dadurch gekennzeichnet, dass** die Reste a, mit denen der Benzolring gemäß Formel (I) substituiert ist, jeweils gleiche Reste sind.

3. Keton nach einem der Ansprüche 1 oder 2, **dadurch gekennzeichnet, dass** die Reste a am Benzolring im Fall von 2 Resten a zueinander in 1,4-Stellung stehen und im Fall von 3 Resten a zueinander in 1, 3, 5-Stellung stehen.

4. Keton nach einem der Ansprüche 1 bis 3, **dadurch gekennzeichnet, dass** der Rest a, jeweils unabhängig voneinander, dem folgendem Rest b entspricht 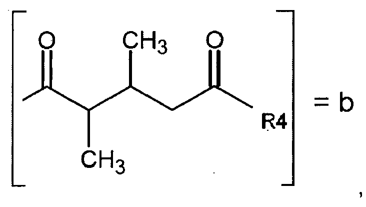 wobei hierbei der Rest R4 für einen ggf. substituierten Kohlenwasserstoffrest mit wenigstens 5 C-Atomen steht, der insbesondere einen cyclischen Kohlenwasserstoffrest umfasst.

5. Keton nach Anspruch 4, **dadurch gekennzeichnet, dass** der Rest b, jeweils unabhängig voneinander, einem der nachfolgenden Reste b1 bis b5 entspricht

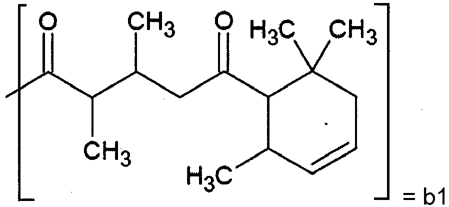

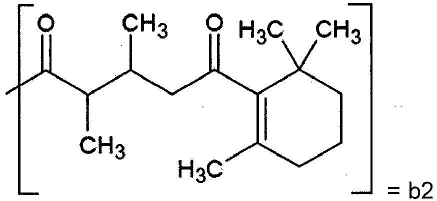

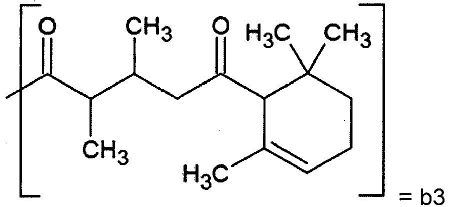

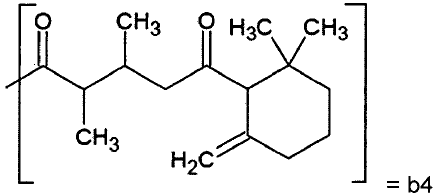

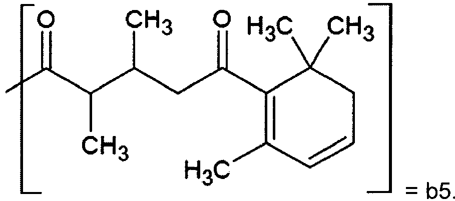

6. Wasch- oder Reinigungsmittel, enthaltend mindestens ein Keton nach einem der Ansprüche 1 bis 4, wobei das genannte Keton vorzugsweise in Mengen zwischen 0,0001 und 5 Gew.-%, vorteilhafterweise zwischen 0,001 und 4 Gew.-%, weiter vorteilhaft zwischen 0,01 und 3 Gew.-%, insbesondere zwischen 0,1 und 2 Gew.-%, jeweils bezogen auf das gesamte Mittel, enthalten ist.

7. Wasch- oder Reinigungsmittel nach Anspruch 6, **dadurch gekennzeichnet, dass** es mindestens ein Tensid ausgewählt aus der Gruppe bestehend aus anionischen, kationischen, nichtionischen, zwitterionischen, amphoteren Tensiden oder Mischungen daraus enthält.

8. Wasch- oder Reinigungsmittel nach einem der Ansprüche 6 oder 7, **dadurch gekennzeichnet, dass** es in fester oder flüssiger Form vorliegt.

9. Verfahren zur lang anhaltenden Beduftung von Oberflächen, **dadurch gekennzeichnet, dass** ein Keton nach einem der Ansprüche 1 bis 5 auf die zu beduftende Oberfläche aufgebracht wird und die genannte Oberfläche anschließend einer elektromagnetischen Strahlung, insbesondere umfassend die Wellenlängen von 200 bis 400 nm, ausgesetzt wird.

## Claims

1. A compound of the general formula (I), 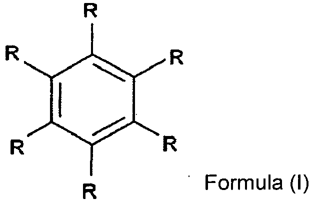 wherein at least two of the functional groups R in formula (I) represent the functional group a shown in square brackets, 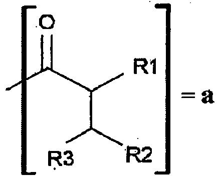
wherein each of the remaining functional groups R in formula (I) represent, independently of one another, hydrogen, a halogen atom, -NO₂, a linear or branched, substituted or unsubstituted alkoxy group having from 1 to 15 C atoms, a linear or branched, substituted or unsubstituted alkyl group having from 1 to 15 C atoms, an aryl functional group, a cycloalkyl functional group, an acyl functional group, -OH, -NH-alkyl, -NH₂ or -N(alkyl)₂, and wherein the following applies for each functional group a, in square brackets, independently of one another:
R2 represents a substituted hydrocarbon functional group having at least one C=O group,
R1 represents a linear or branched, substituted or unsubstituted alkyl group having from 1 to 6 C atoms, in particular a methyl group, and
R3 represents hydrogen, a halogen atom, -NO₂, a linear or branched, substituted or unsubstituted alkoxy group having from 1 to 15 C atoms, a linear or branched, substituted or unsubstituted alkyl group having from 1 to 15 C atoms, an aryl functional group, a cycloalkyl functional group, an acyl functional group, -OH, -NH-alkyl, -NH₂ or -N(alkyl)₂.

2. A ketone according to claim 1, **characterized in that** the functional groups a, by which the benzene ring according to formula (I) is substituted, are identical functional groups in each case.

3. The ketone according to one of claims 1 or 2, **characterized in that** the functional groups a in the benzene ring are positioned relative to one another at the 1,4 position when there are two functional groups a and are positioned relative to one another at the 1,3,5 position when there are three functional groups a.

4. The ketone according to one of claims 1 to 3, **characterized in that** each of the functional groups a corresponds, independently of one another, to the following functional group b, 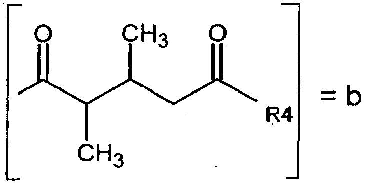 where, in this case, functional group R4 represents an optionally substituted hydrocarbon functional group having at least 5 C atoms, which comprises in particular a cyclic hydrocarbon functional group.

5. The ketone according to claim 4, **characterized in that** each of the functional groups b corresponds, independently of one another, to one of the following functional groups b1 to b5

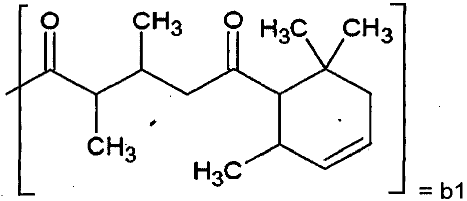

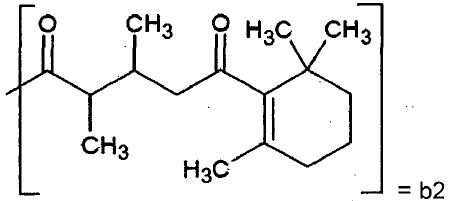

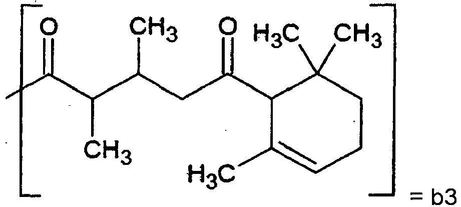

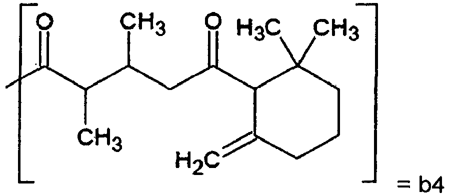

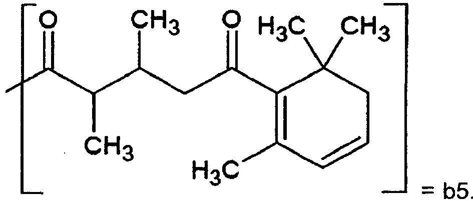

6. A washing or cleaning agent containing at least one ketone according to one of claims 1 to 4, wherein said ketone is preferably contained in amounts of between 0.0001 and 5 wt.%, advantageously between 0.001 and 4 wt.%, more advantageously between 0.01 and 3 wt.%, and in particular between 0.1 and 2 wt.%, based on the overall agent in each case.

7. The washing or cleaning agent according to claim 6, **characterized in that** it contains at least one surfactant selected from the group consisting of anionic, cationic, non-ionic, zwitterionic or amphoteric surfactants, or mixtures thereof.

8. The washing or cleaning agent according to one of claims 6 or 7, **characterized in that** it is present in solid or liquid form.

9. A method for fragrancing surfaces in a lasting manner, **characterized in that** a ketone according to one of claims 1 to 5 is applied to the surface to be fragranced and said surface is then exposed to electromagnetic radiation, in particular having wavelengths of from 200 to 400 nm.

## Revendications

1. Composé de la formule générale (I), 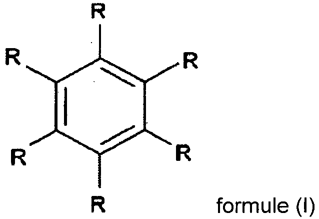 au moins deux des radicaux R de la formule (I) représentant le radical a placé entre crochets, 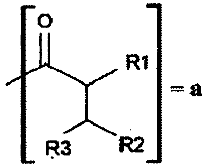
et les autres radicaux R de la formule (I), représentant chacun, indépendamment l'un de l'autre, un atome d'hydrogène, un atome d'halogène, -NO₂, un groupe alcoxy linéaire ou ramifié, substitué ou non substitué, ayant 1 à 15 atomes de carbone, un groupe alkyle linéaire ou ramifié, substitué ou non substitué, ayant 1 à 15 atomes de carbone, un groupe aryle, un radical cycloalkyle, un radical acyle, -OH, -NH-alkyle, -NH₂ ou -N(alkyle)2,
et pour chaque radical a placé entre crochets pour chaque résidu, indépendamment,
R2 représente un radical hydrocarboné substitué qui comporte au moins un groupe C=O-, R1 représente un groupe alkyle linéaire ou ramifié, substitué ou non substitué, ayant 1 à 6 atomes de carbone, en particulier un groupe méthyle, et
R3 représente l'hydrogène, un atome d'halogène, -NO₂, un groupe alcoxy linéaire ou ramifié, substitué ou non substitué, ayant 1 à 15 d'atomes de carbone, un groupe alkyle linéaire ou ramifié, substitué ou non substitué, ayant 1 à 15 atomes de carbone, un radical aryle, un radical cycloalkyle, un radical acyle, -OH, -NH-alkyle, -NH₂ ou -N(alkyle)₂.

2. Cétone selon la revendication 1, **caractérisée en ce que** les radicaux a, avec lesquels le noyau benzénique de la formule (I) est substitué, sont des radicaux identiques.

3. Cétone selon l'une des revendications 1 ou 2, **caractérisée en ce que** les radicaux a sur le noyau benzénique sont, dans le cas de deux radicaux a, en positions 1,4 l'un par rapport à l'autre et, dans le cas de trois radicaux, en positions 1, 3, 5 les uns par rapport aux autres.

4. Cétone selon l'une des revendications 1 à 3, **caractérisée en ce que** le radical a correspond, indépendamment, au radical b suivant. 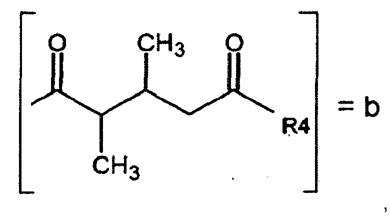 le radical R4 représentant un radical hydrocarboné éventuellement substitué, ayant au moins 5 atomes de carbone, qui comprend en particulier un radical hydrocarboné cyclique.

5. Cétone selon la revendication 4, **caractérisée en ce que** le radical b correspond, indépendamment, à l'un des radicaux b1 à b5

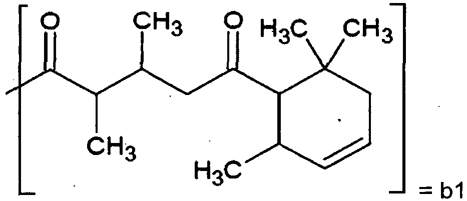

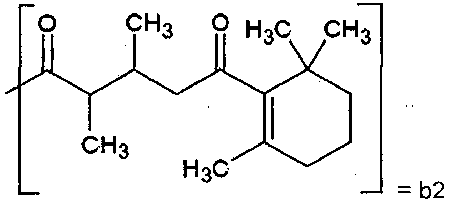

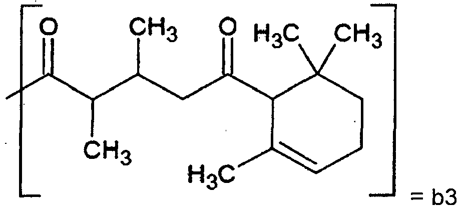

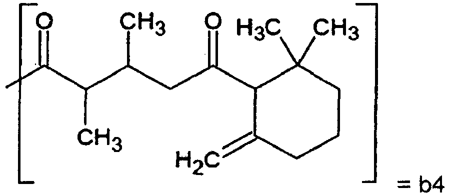

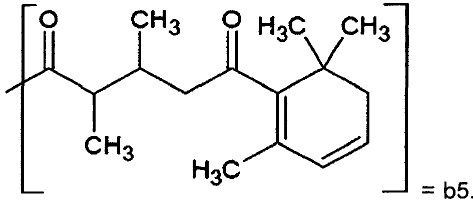

6. Agent de lavage ou de nettoyage contenant au moins une cétone selon l'une des revendications 1 à 4, dans lequel ladite cétone est contenue avantageusement dans des quantités comprises entre 0,0001 et 5% en poids, de préférence entre 0,001 de 4% en poids, plus préférablement entre 0,01 et 3% en poids, notamment entre 0, 1 et 2% en poids, à chaque fois par rapport à tout l'agent.

7. Agent de lavage ou de nettoyage selon la revendication 6, **caractérisé en ce qu'**il contient au moins un tensioactif choisi dans le groupe constitué par les tensioactifs anioniques, cationiques, non ioniques, zwitterioniques, amphotères ou leurs mélanges.

8. Agent de lavage ou de nettoyage selon l'une des revendications 6 ou 7, **caractérisé en ce qu'**il se présente sous forme solide ou liquide.

9. Procédé pour parfumer sur une longue durée des surfaces, **caractérisé en ce qu'**une cétone selon l'une des revendications 1 à 5 est appliquée à la surface à parfumer et ladite surface est exposée ensuite à un rayonnement électromagnétique, comprenant en particulier les longueurs d'onde allant de 200 et 400 nm.
